# EUROPÄISCHE PATENTSCHRIFT

(11) **EP 1 477 166 B1**
(45) Veröffentlichungstag und Bekanntmachung des Hinweises auf die Patenterteilung: **23.08.2006**
(21) Anmeldenummer: 03009559.0
(22) Anmeldetag: 28.04.2003
(51) Int. Cl.: A61K 31/428, A61K 45/06, A61P 17/06, A61P 17/00

(54) **Verwendung von Riluzole kombiniert mit geeigneten Hilfs-und Zusatzstoffen zur Behandlung von Krankheiten, die durch eine Hyperproliferation von Keratinozyten gekennzeichnet sind, insbesondere Neurodermitis und Psoriasis**
The use of riluzol combined with excipients and additives for the treatment of disorders characterised by hyperproliferation of keratinocytes, in particular neurodermitis and psoriasis
Utilisation du rilusole combiné avec des excipients et des additifs pour le traitement de troubles caractérisés par une hyperprolifération de kératinocytes, notamment la névrodermite et le psoriasis

(43) Veröffentlichungstag der Anmeldung: 17.11.2004
(73) Patentinhaber: Biofrontera Bioscience GmbH, 51377 Leverkusen (DE)
(72) Erfinder: Sych, Michael, 81371 München (DE); Goppelt, Andreas, 80636 München (DE)
(74) Vertreter: Zwicker, Jörk

(56) Entgegenhaltungen:
- WO-A-03/016563
- ABDUL M ET AL: "Voltage-gated sodium ion channels in prostate cancer: Expression and activity" ANTICANCER RESEARCH 2002 GREECE, Bd. 22, Nr. 3, 2002, Seiten 1727-1730, XP008021818 ISSN: 0250-7005

## Beschreibung

Die Erfindung betrifft die Verwendung von Riluzol gegebenenfalls mit geeigneten Hilfs- und Zusatzstoffen zur Herstellung eines Arzneimittels zur Behandlung von Psoriasis Neurodermitis, aktinische Keratose, Hyper Keratosen, insbesondere epidermolytische Hyper Keratosen, Hyper Keratosis Lenticularis Perstans, Keratosis pilaris und Ichthyose sowie Zusammensetzungen enthaltend Riluzol und deren Verwendung.

### Stand der Technik

Riluzol (2-Amino-6-(trifluormethoxy)-benzothiazol) der folgenden Formel: 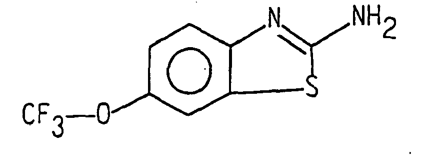 ist eine antikonvulsive Substanz (Stutzmann et al., 1991, J. Pharmacol., 193: 223-229) mit anästhesierenden Eigenschaften bei hohen Konzentrationen (Mantz et al., 1992, Anesthesiology, 76: 844-848). Sie wirkt neuroprotektiv sowohl bei fokaler als auch globaler Ischämie (z.B. Malgouris et al., 1989, J. Neurosci., 9: 3720-3727). Weiterhin wirkt die Substanz sedativ und ist insbesondere geeignet um Rückenmarksverletzungen zu verhindern (Lang-Lazdunsky et al., 1999, J. Thorac Cardiovasc Surg, 117: 881-889; Romettino et al., 1991, Eur J Pharm, 199: 371-373). Außerdem wirkt Riluzol angstlösend (US 4,370,338). Die Substanz wird erfolgreich zur Therapie von amyothropher Lateralsklerose (ALS) eingesetzt und verlangsamt dabei das Fortschreiten der Erkrankung (Bryson et al., 1996, Drug Eval., 52: 549-563). Riluzol wurde *in vivo* auch für andere neurodegenerative Erkrankungen erfolgreich im Tiermodell getestet (Barneoud et al., 1996, Neuroscience, 74: 971-983; Mary et al., 1995, Neurosci Lett 1: 92-96).

Auf molekularer Ebene ist der Wirkmechanismus nicht völlig geklärt. In hohen Konzentrationen inhibiert Riluzol einige der postsynaptischen Effekte von Glutamat (Doble, 1996, Neurology 47 (suppl. 4), 5: S233-S241), jedoch wird die positive Wirkung von Riluzol hauptsächlich der Wirkung zugeschrieben, die glutaminerge Übertragung durch Inhibition der Glutamatfreisetzung zu inhibieren (Doble, supra). Diese Wirkung wiederum ist möglicherweise zum Teil auf die inhibitorische Wirkung von Riluzol auf spannungsabhängige Natriumkanäle, aber möglicherweise auch auf die aktivatorische Wirkung von Riluzol auf Two P domain potassium channels (TREK-1, TRAAK) zurückzuführen (Duprat et al., Mol. Pharmacol., 2000, 57: 906-912; Benoit und Escande, 1991, Pflügers Arch 419: 603-609). Riluzol wirkt jedoch auch auf eine Vielzahl anderer Ionenkanäle, z.B. inhibitorisch auf Herz-Na⁺-Kanäle (Mestre et al., Fundam. Clin. Pharmacol., 2000, 14: 107-117), Riluzol interferiert mit NMDA-vermittelten Antworten über einen Pertussistoxin-sensitiven Mechanismus (Hubert et al., Br. J. Pharmacol., 1994, 113: 261-267), aktiviert large-conductance calciumactivated potassium channels (Wu und Li, J. Investig. Med., 1999, 484-495), inhibiert sowohl Hoch- und Niedrigspannung-aktivierte Calcium-Ströme (Stefani et al., Exp. Neurol., 1997, 147: 115-122), verlangsamt die Inaktivierung spannungsabhängiger Kv1.4-Kaliumkanäle (Xu et al., J. Pharmacol. Exp. Ther., 2001, 299: 227-237) und aktiviert SK3 Kaliumkanäle (Grunnet et al., Neuropharmacology, 2001, 40: 879-887).

### Erfindung

Überraschenderweise wurde nun gefunden, daß Riluzol erfolgreich die Proliferation von Keratinozyten hemmen kann und daher überraschenderweise geeignet ist, um gegebenenfalls kombiniert mit geeigneten Hilfs- und Zusatzstoffen, die Krankheiten Psoriasis, Neurodermitis, aktinische Keratose, Hyperkeratosen wie epidermolytische Hyperkeratose, Hyperkeratosis Lenticularis Perstans sowie Keratosis pilaris und Ichthyosen, die durch eine Hyperproliferation von Keratinozyten gekennzeichnet sind, zu behandeln und/oder deren Ausbruch zu verhindern. Bevorzugte Erkrankungen sind Neurodermitis und Psoriasis, insbesondere Psoriasis.

Die Erfindung betrifft daher die Verwendung von Riluzol oder eines pharmazeutisch annehen Salzes davon zur Herstellung eines Arzneimittels zur Therapie oder Prävention von Psoriasis, Neurodermitis, aktinische Keratose, Hyperkeratosen wie epidermolytische Hyperkeratose, Hyperkeratosis Lenticularis Perstans sowie Keratosis pilaris und Ichthyosen die durch Hyperproliferation von Keratinozyten gekennzeichnet sind. Gegebenenfalls kann Riluzol oder ein pharmazeutisch annehmbares Salz davon mit geeigneten Hilfs- und Zusatzstoffen kombiniert werden.

Die Erkrankung ist ausgewählt aus Psoriasis, Neurodermitis, aktinischer Keratose, Hyperkeratosen wie epidermolytische Hyperkeratose, Hyperkeratosis Lenticularis Perstans, Keratosis pilaris und Ichthyosen.

Besonders bevorzugte Erkrankungen sind dabei Psoriasis und Neurodermitis, insbesondere Psoriasis.

### Krankheiten die durch eine Hyperproliferation von Keratinozyten gekennzeichnet sind

Im Sinne der vorliegenden Erfindung sind Krankheiten, die durch eine Hyperproliferation von Keratinozyten gekennzeichnet sind, Krankheiten bei denen die Patienten lokal oder am ganzen Körper eine im Vergleich zu gesunder Epidermis verdickte Epidermis aufweisen. Als eine verdickte Epidermis gilt eine Epidermis die im Vergleich zu gesunder Haut um mindestens ca. 10%, vorzugsweise ca. 30%, insbesondere ca. 50%, am meisten bevorzugt ca. 80% und mehr verdickt ist. Dem Fachmann sind Verfahren zur Messung der Epidermisdicke bekannt. Beispielsweise beschreiben Wetzel et al. (Arch. Dermatol. Res., April 2003) mit der optischen Kohärenz Tomographie und Baulieu et al. (Proc. Natl. Acad. Sci. USA, 2000, 97:4279-4284) mit Hautechographie, die ein nicht-invasives Verfahren zur Messung der Dicke der Epidermis darstellt. Weiterhin kann die Dicke der Epidermis histologisch in Schnitten von Hautbiopsien bestimmt werden, wie beispielsweise in El-Domyati et al. (Exp. Dermatol. 2002; 11:398-405) oder Schopf et al. (J. Am. Acad. Dermatol. 2002; 46:886-91) beschrieben. Da die Epidermis in verschiedenen Hautregionen unterschiedlich dick ist, müssen beim Vergleich der Dicken gesunder und erkrankter Epidermis jeweils die Epidermisdicken in gleichen Hautregionen verglichen werden. Darüberhinaus gibt es noch eine gewisse Variation der Epidermisdicke der selben Hautregion zwischen zwei Individuen. Daher ist es bevorzugt, daß die Dicke der Epidermis beispielsweise am linken und am rechten Bein eines erkrankten Individuums gemessen werden unter der Voraussetzung, daß nicht die gesamte Haut von der Erkrankung betroffen ist. In der Regel gehen Erkrankungen, die durch eine Hyperproliferation von Keratinozyten gekennzeichnet sind, mit einer Rötung der betreffenden Hautbereiche einher, so daß der Fachmann erkrankte Hautbereiche eines Patienten allein an der Rötung von gesunden Hautbereichen unterscheiden kann. Die Verdickung der Epidermis bei Krankheiten, die durch eine Hyperproliferation von Keratinozyten gekennzeichnet sind, kann beispielsweise nur lokal auftreten, oder, wie bei Psoriasis, bereits in Haut von Psoriasispatienten nachweisbar sein, die nicht auf Grund einer Rötung bzw. einer Läsion als betroffen erkennbar ist. Jedoch ist auch bei Psoriasispatienten eine weitere Verdickung der Epidermis in betroffenen Hauarealen (=Läsionen) zu beobachten. Beispiele für Erkrankungen, die durch eine Hyperproliferation von Keratinozyten im Sinne der vorliegenden Erfindung gekennzeichnet sind, sind Psoriasis, Neurodermitis, aktinische Keratose, Hyperkeratosen wie epidermolytische Hyperkeratose und Hyperkeratosis Lenticularis Perstans, sowie Keratosis pilaris und Ichthyosen. Besonders bevorzugte Erkrankungen im Sinne der vorliegenden Erfindung sind Neurodermitis und Psoriasis, insbesondere Psoriasis.

Die Epidermis wird hauptsächlich aus Keratinozyten gebildet, die von der Basalschicht langsam nach außen wandern. Dabei gehen sie von einem proliferierenden in ein differenzierenden Status über, um am Ende abzusterben. Die abgestorbenen Keratinozyten bilden dann an der Oberfläche der Haut die Hornschicht, von der ständig tote Zellen abgestoßen werden. Durch diesen Prozeß kommt es ständig zu einer Erneuerung der Haut. In Krankheiten, die durch eine Hyperproliferation der Keratinozyten gekennzeichnet sind, ist die Balance zwischen Differenzierung und Proliferation der Keratinozyten zugunsten der Proliferation verschoben, wodurch sich die Epidermis, die mehr Keratinozyten, insbesondere proliferierende Keratinozyten enthält, stark verdickt. In solchen Erkrankungen findet man auch häufig gestörte Barrierefunktionen wodurch Superantigene oder Pathogene leichter in die Haut eindringen können. Häufig wird auch eine verstärkte Inflammation beobachtet, wie z.B. bei Neurodermitis und Psoriasis, die dann mit der bereits erwähnten Rötung der Haut einhergeht.

Gegenwärtig existieren nur unbefriedigende Therapien zur Behandlung dieser Krankheiten, die oft nur bei einer Subpopulation von Patienten erfolgreich ist, und bestehende Therapien wie die topische oder systemische Applikation von Cortikosteroiden oder Cyclosporin bei Neurodermitis oder Psoriasis sind häufig mit starken Nebenwirkungen und der Unterdrückung des Immunsystems verbunden. Es besteht daher ein Bedürfnis nach neuen möglichst nebenwirkungsfreien Medikamenten zur Behandlung dieser Erkrankungen. Das erfindungsgemäß verwendbare Riluzol stellt ein solches Medikament dar. Beispielsweise wurden bei der systemischen Gabe von bis zu 100 mg Riluzol zur Therapie der Laterosklerose, nur relativ leichte Nebenwirkungen beobachtet von denen die schwersten eine Asthenie (18% der Behandelten) und Brechreiz (16% aller Behandelten) waren, die zudem bei längeranhaltender Therapie über beispielsweise 6 Monate deutlich verringert wurden. Darüberhinaus eignet sich Riluzol aufgrund seiner Lipophilität zur topischen Applikation wodurch sich die Nebenwirkungen noch weiter verringern lassen.

Erfindungsgemäß verwendbare Arzneimittel können dabei zur Behandlung von lokalen Läsionen, aber auch zur Prävention des Ausbruchs der Krankheit eingesetzt werden. So kann beispielsweise durch frühzeitige Behandlung von Psoriasispatienten ohne Läsionen ein Ausbruch der Krankheit mit dermatologischen Manifestationen verhindert werden, indem durch eine Gabe von Riluzol eine weitere Verdickung der Epidermis verhindert wird.

### Pharmazeutisch annehmbare Salze

Das erfindungsgemäß verwendbare Riluzol kann in jeder beliebigen Form zur Verfügung gestellt werden die für eine Verabreichung geeignet ist. Geeignete pharmazeutisch annehmbare Formen umfassen Salze.

Beispiele pharmazeutisch annehmbarer Salze umfassen, ohne darauf beschränkt zu sein, nichttoxische anorganische und organische Salze wie Azetat, abgeleitet von Essigsäure, Aconat abgeleitet von Aconitinsäure, Ascorbat abgeleitet von Ascorbinsäure, Benzosulfonate abgeleitet von Benzosulfonsäure, Benzoat abgleitet von Benzoesäure, Cinnamat abgeleitet von Zimtsäure, Citrat abgeleitet von Zitronensäure, Embonat abgeleitet von Embonsäure, Enantat abgeleitet von Heptansäure, Formiat abgeleitet von Ameisensäure, Fumarat abgeleitet von Fumarsäure, Glutamat abgeleitet von Glutaminsäure, Glykolat abgleitet von Glykolsäure, Chlorid abgleitet von Salzsäure, Bromid abgeleitet von Bromwasserstoff, Laktat abgeleitet von Milchsäure, Maleat abgeleitet von Maleinsäure, Malonat abgeleitet von Malonsäure, Mandelat abgeleitet von Mandelsäure, Methansulfonat abgeleitet von Methansulfonsäure, Naphtalin-2 -Sulfonat abgeleitet von Naphtalin-2-sulfonsäure, Nitrat abgeleitet von Salpetersäure, Perchlorat abgeleitet von Perchlorsäure, Phosphat abgeleitet von Phosphorsäure, Phtalat abgeleitet von Phtalsäure, Salicylat abgeleitet von Salicylsäure, Sorbat abgeleitet von Sorbinsäure, Stearat abgeleitet von Stearinsäure, Succinat abgeleitet von Succinsäure, Sulfat abgeleitet von Schwefelsäure, Tartrat abgeleitet von Weinsäure, Toluol-p-sulfonat abgeleitet von p-Tuoluol-sulfonsäure and andere. Solche Salze können durch dem Fachmann bekannte und im Stand der Technik beschriebene Verfahren hergestellt werden. Andere Salze wie Oxalat abgeleitet von Oxalsäure, die nicht als pharmazeutisch annehmbar in Betracht gezogen werden, können als Zwischenprodukte zur Herstellung von Riluzol oder eines pharmazeutisch annehmbaren Salzes davon geeignet sein.

### Formulierungen

Der Begriff "Hilfsstoff" bedeutet erfindungsgemäß jedes, pharmazeutische annehmbare festes oder flüssiges Füll-, Verdünnungs-, oder Verpackungsmaterial, solange es nicht nachteilhaft mit Riluzol oder dem pharmazeutisch annehmbaren Salz davon reagiert. Flüssige galenische Hilfsstoffe sind zum Beispiel steriles Wasser, physiologische Kochsalzlösung, Zuckerlösungen, Ethanol und/oder Öle. Galenische Hilfsstoffe zur Herstellung von Tabletten und Kapseln können zum Beispiel Bindemittel und Füllmaterial enthalten.

Die Herstellung von Arzneimitteln enthaltend Riluzol bzw. dessen Einsatz bei der erfindungsgemäßen Verwendung erfolgt in üblicher Weise anhand geläufiger pharmazeutisch-technologischer Verfahren. Hierzu wird Riluzol mit geeigneten, pharmazeutisch annehmbaren Hilfs- und Trägerstoffen zu den für die verschiedenen Indikationen und für den jeweiligen Applikationsort geeigneten Arzneiformen verarbeitet. Dabei können die Arzneimittel in einer Weise hergestellt werden, daß die jeweils erwünschte Freisetzungsrate, z.B. eine rasche Anflutung und/oder ein Retard- bzw. Depoteffekt erzielt werden.

In einer besonders bevorzugten Verwendung der vorliegenden Erfindung wird das oben beschriebene Arzneimittel zur Therapie oder Prävention von Erkrankungen, die durch Hyperproliferation von Keratinozyten gekennzeichnet sind, topisch appliziert.

Zur topischen Applikation auf der Haut, einer Wunde oder einer Schleimhaut wird das Riluzol enthaltende Arzneimittel vorzugsweise in Form einer Emulsion, eines Gels, einer Salbe, eines Schaums, eines Pflasters, einer Creme eines mischphasige bzw. amphiphile Emulsionssystems (Öl/ Wasser-Wasser/Öl-Mischphase), eines Liposoms oder Transfersoms zubereitet. Diese Zubereitungsformen sind im Stand der Technik bekannt und Riluzol kann vom Fachmann ohne weiteres zu einem Arzneimittel in diese Zubereitungsformen verarbeitet werden.

Weitere topisch applizierbare Formen sind Puder, Pasten, oder Lösungen. Die Pasten enthalten als konsistenzgebende Grundlagen oft lipophile und hydrophile Hilfsstoffe mit sehr hohem Feststoffanteil. Die Puder, insbesondere topisch applizierbare Puder können zur Erhöhung der Dispersität sowie des Fließ- und des Gleitvermögens sowie zur Verhinderung von Agglomeraten, Stärken, wie Weizen- oder Reisstärke, flammendisperse Siliziumdioxide und/oder Kieselerden enthalten. Diese Zusatzstoffe können auch als Verdünnungsmittel dienen.

In einer bevorzugten Ausführungsform der vorliegenden Erfindung wird daher das Riluzol enthaltende Arzneimittel, das zur Therapie oder Prävention von Erkrankungen, die durch Hyperproliferation von Keratinozyten gekennzeichnet sind, als eine Salbe, ein Gel, ein Pflaster, eine Emulsion, eine Lotion, ein Schaum, eine Creme der mischphasigen oder amphiphile Emulsionssysteme (Öl/Wasser-Wasser/Öl-Mischphase), ein Liposom, ein Transfersom, eine Paste oder ein Puder zubereitet.

Als Hilfs- bzw. Trägerstoffe insbesondere für die Zubereitung topisch applizierbarer Arzneimittel der vorliegenden Erfindung eignet sich beispielsweise Natriumalginat als Gelbildner zur Herstellung einer geeigneten Grundlage oder Cellulosederivate, wie z. B. Guar- oder Xanthangummi, anorganische Gelbildner, wie z.B. Aluminiumhydroxide oder Bentonite (sog. thixotrope Gelbildner), Polyacrylsäurederivate, wie z.B. Carbopol®, Polyvinylpyrrolidon, mikrokristalline Cellulose oder Carboxymethylcellulose, beispielsweise das Carboxymethylcellulose-Produkt IntraSite (Smith & Nephew, London). Darüber hinaus können biokompatible Poloxamere verwendet werden, wie beispielsweise FloGel®, das ein thermoreversibles Gel bildet. Darüber hinaus kommen Phospholipide oder amphiphile nieder- und höhermolekulare Verbindungen in Frage. Die Gele können entweder als Hydrogele auf Wasserbasis oder als hydrophobe Organogele, beispielsweise auf Basis von Gemischen nieder- und hochmolekularer Paraffinkohlenwasserstoffe und Vaseline vorliegen. Weiterhin können synthetische Biomaterialien als Trägerstoffe eingesetzt werden, wobei Riluzol nichtkovalent oder kovalent, beispielsweise direkt oder über einen Linker gebunden sein kann.

Dem Fachmann bekannte hautberuhigende und/oder endzündungshemmende Zusatzstoffe wie beispielsweise synthetisch hergestellte Wirkstoffe und/oder Extrakte und/oder Wirkstoffe aus Heilpflanzen, insbesondere Bisobolol und Panthenol können dem Arzneimittel ebenfalls zugesetzt werden. Des weiteren können Farbstoffe beispielsweise gelbes und/oder rotes Eisenoxid und/oder Titandioxid zur farblichen Anpassung und/oder Geruchsstoffe dem Arzneimittel hinzugesetzt werden.

Des weiteren können die erfindungsgemäß verwendbaren Arzneimittel Emulgatoren umfassen. Als Emulgatoren, eignen sich neutrale, anionische oder kationische Tenside, beispielsweise Alkaliseifen, Metallseifen, Aminseifen, sulfurierte und sulfonierte Verbindungen, Invertseifen, lange Fettalkohole, Partialfettsäureester des Sorbitans und Polyoxyethylensorbitans, z.B. Lanette-Typen, Wollwachs, Lanolin oder andere synthetische Produkte, die zur Herstellung von Öl/Wasser- und/oder Wasser/Öl-Emulsionen geeignet sind. Die hydrophilen Organogele können beispielsweise auf Basis hochmolekularer Polyethylenglykole zubereitet werden. Diese gelartigen Formen sind abwaschbar. Als Lipide in Form fett- und/oder öl- und/oder wachsartiger Komponenten zur Herstellung der Salben, Cremes oder Emulsionen werden Vaseline, natürliche und/oder synthetische Wachse, Fettsäuren, Fettalkohole, Fettsäureester, z.B. als Mono-, Di- oder Triglyceride, Paraffinöl oder pflanzliche Öle, gehärtetes Rizinusöl oder Kokosöl, Schweinefett, synthetische Fette, z.B. auf Capryl-, Caprin-, Laurin- und Stearinsäurebasis wie z.B. Softisan® oder Triglyceridgemischen wie Miglyol® eingesetzt.

Zur Einstellung des pH-Wertes können weiterhin beispielsweise osmotisch wirksame Säuren und Laugen, z.B. Salzsäure, Zitronensäure, Natronlauge, Kalilauge, Natriumhydrogencarbonat, ferner Puffersysteme, wie z.B. Citrat, Phosphat, Tris-Puffer oder Triethanolamin verwendet werden. Des weiteren kann die Stabilität durch hinzufügen von Konservierungsmittel, wie z.B. Methyl- oder Propylbenzoat (Parabene) oder Sorbinsäure erhöht werden.

Zur nasalen Applikation können Nasentropfen oder Nasensprays Vernebler oder Nasencreme oder -salbe eingesetzt werden. Nasenspray oder Trockenpulverzubereitungen sowie Dosieraerosole eignen sich darüber hinaus auch zur systemischen Verabreichung von Riluzol oder einem pharmazeutisch annehmbaren Salz davon. Weiterhin können die erfindungsgemäßen Arzneimittel über Druck- bzw. Dosieraerosole und Trockenpulverzubereitungen inhaliert bzw. insuffliert werden. Derartige Verabreichungformen können auch für die direkte, regionale Applikation in der Lunge, en Bronchien und/oder dem Kehlkopf bzw. für die lokale Applikation verwendet werden. Dabei können die Trockenpulverzusammensetzungen beispielsweise als Wirkstoff-Softpellets, als Wirkstoff-Pulvermischungen mit geeigneten Trägerstoffen, wie z.B. Lactose und/oder Glukose formuliert werden. Für die Inhalation oder Insufflation eignen sich übliche, dem Fachmann bekannte Applikatoren, die sich zur Behandlung des Nasen-, Mund- und/oder Rachenraums eignen. Riluzol oder ein pharmazeutisch annehmbares Salz davon lässt sich auch mittels eines Ultraschallvemebelungsgerätes applizieren. Anstelle der Dosieraerosole lassen sich auch treibgasfreie, manuelle Pumpsysteme verwenden. Zweckmäßigerweise können die Treibgasaerosole auch oberflächenaktive Hilfsstoffe enthalten, wie z.B. Isopropylmyristat, Polyoxyethylensorbitantfettsäureester, Lecithine oder Sojalecithin. Für die regionale Applikation *in situ* sind z.B. Lösungen zur Instillation geeignet.

Weiterhin kann Riluzol oder ein pharmazeutisch annehmbares Salz davon in Form von systemisch eingesetzten Arzneimitteln verwendet werden. Dazu gehören die Parenteralia, zu denen unter anderem die Injektabilia und Infusionen gehören. Injektabilia werden entweder in Form von Ampullen oder auch als sog. gebrauchsfertige Injektabilia, z.B. als Fertigspritzen oder Einmalspritzen, daneben auch in Durchstechflaschen zur mehrmaligen Entnahme hergerichtet. Die Verabreichung der Injektabilia kann in Form einer subkutanen (s.c.), intramuskulären (i.m.), intravenösen (i.v.) oder intrakutanen (i.c.) Applikation erfolgen. Insbesondere können die jeweils zweckmäßigen Injektionsformen als Kristallsuspensionen, Lösungen, nanopartikuläre oder kolloiddisperse Systeme, wie z.B. Hydrosole, hergestellt werden.

Die injizierbaren Zubereitungen können ferner als Konzentrate hergestellt werden, die mit wässrigen isotonischen Verdünnungsmitteln aufgelöst oder dispergiert werden können. Die Infusionen lassen sich ebenfalls in Form von isotonischen Lösungen, Fettemulsionen, Liposomenzubereitungen, Mikroemulsionen, zubereiten. Wie Injektabilia können auch Infusionszubereitungen in Form von Konzentraten zum Verdünnen zubereitet werden. Die injizierbaren Zubereitungen können auch in Form von Dauerinfusionen sowohl in der stationären als auch in der ambulanten Therapie, z.B. in Form von Minipumpen, appliziert werden.

Den parenteralen Arzneiformen können beispielsweise Albumin, Plasmaexpander, oberflächenaktive Stoffe, organische Lösungsmittel, pH-beeinflussenden Stoffe, komplexbildende Stoffe oder polymere Stoffe, insbesondere als Substanzen zur Beeinflussung der Adsorption von Riluzol oder eines pharmazeutisch annehmbaren Salzes davon an Proteine oder Polymere oder auch mit dem Ziel hinzugefügt werden, die Adsorption von Riluzol oder eines pharmazeutisch annehmbaren Salzes davon an Materialien, wie Injektionsbestecke oder Verpackungsmittel, beispielsweise Kunststoff oder Glas, zu verringern.

Riluzol oder ein pharmazeutisch annehmbares Salz davon kann in den Parenteralia an Microcarrier oder Nanopartikel gebunden sein, beispielsweise an feinstverteilte Partikel auf Basis von Poly(meth)acrylaten, Polylactaten, Polyglycolaten, Polyaminsäuren oder Polyetherurethanen. Die parenteralen Zubereitungen können auch als Depotpräparate modifiziert sein, z.B. aufbauend auf dem "Multiple Unit Prinzip", wenn Riluzol oder ein pharmazeutisch annehmbares Salz davon in feinstverteilter bzw. dispergierte, suspendierter Form oder als Kristallsuspension in das Arzneimittel eingearbeitet ist oder aufbauend auf dem "Single Unit Prinzip", wenn Riluzol oder ein pharmazeutisch annehmbares Salz davon in einer Arzneiform, z.B. ein Tablette oder ein Stäbchen, eingeschlossen ist das anschließend implantiert wird. Häufig bestehen diese Implantate oder Depotarzneimittel bei Single Unit- und Multiple Unit-Arzneiformen aus sogenannten bioabbaubaren Polymeren, wie z.B. Polyestern der Milch- und Glykolsäure, Polyetherurethanen, Polyaminosäuren, Poly(meth)acrylaten oder Polysacchariden.

Als Hilfs- und Trägerstoffe bei der Herstellung von als Parenteralia formulierten erfindungsgemäß verwendbaren Arzneimitteln werden vorzugsweise Aqua sterilisata, den pH-Wert beeinflussende Substanzen, wie z.B. organische und anorganische Säuren und Basen sowie deren Salze, Puffersubstanzen zur Einstellung des pH-Wertes, Isotonisierungsmittel, wie z.B. Natriumchlorid, Natriumhydrogencarbonat, Glucose und Fructose, Tenside bzw. oberflächenaktive Substanzen und Emulgatoren, wie z.B. Partialfettsäureester des Polyoxyethylensorbitans (beispielsweise Tween®) oder z.B. Fettsäureester des Polyoxyethylens (beispielsweise Cremophor®), fette Öle, wie z.B. Erdnussöl, Sojabohnenöl und Rizinusöl, synthetische Fettsäureester, wie z.B. Ethyloleat, Isopropylmyristat und Neutralöl (beispielsweise Miglyol®), sowie polymere Hilfsstoffe wie z.B. Gelatine, Dextran, Polyvinylpyrrolidon, die Löslichkeit erhöhende Zusätze organischer Lösungsmittel wie z.B. Propylenglycol, Ethanol, N,N-Dimethylacetamid, Propylenglycol oder komplexbildender Stoffe, wie z.B. Citraten und Harnstoff, Konservierungsmittel, wie z.B. Benzoesäurehydroxypropylester und -methylester, Benzylalkohol, Antioxidantien, wie z.B. Natriumsulfit und Stabilisatoren, wie z.B. EDTA, zugesetzt.

Bei Zubereitung des erfindungsgemäß verwendbaren Arzneimittels als Suspensionen erfolgt in einer bevorzugten Ausführungsform ein Zusatz von Verdickungsmitteln um das Absetzen des Riluzol oder eines pharmazeutisch annehmbaren Salzes davon zu verhindern, von Tensiden und Peptisatoren, um die Aufschüttelbarkeit des Sediments zu sichern, und/oder von Komplexbildnern wie beispielsweise EDTA. Auch lassen sich mit verschiedenen Polymeren Wirkstoffkomplexe erzielen. Beispiele solcher Polymere sind Polyethylenglykol, Polystyrol, Carboxymethylcellulose, Pluronics® oder Polyethylenglykolsorbitfettsäureester. Riluzol oder ein pharmazeutisch annehmbares Salz davon lässt sich auch in Form von Einschlussverbindungen, z.B. mit Cyclodextrinen, in flüssige Zubereitungen inkorporieren. Als weitere Hilfsstoffe können in bestimmten Ausführungsformen auch Dispergiermittel zugesetzt werden. Zur Herstellung von Lyophilisaten können Gerüstbildner, wie z.B. Mannit, Dextran, Saccharose, Humanalbumin, Lactose, PVP oder Gelatinesorten verwendet werden.

Soweit Riluzol nicht in Form seiner Base in die flüssigen Arzneizubereitungen eingearbeitet wird, lässt es sich in Form seiner Säureadditionssalz-Solvate in den Parenteralia einsetzen.

Eine weitere systemische Applikationsform die von Bedeutung ist, ist die perorale Verabreichung als Tabletten, Hart- oder Weichgelatinekapseln, Dragees, Pulver, Pellets, Mikrokapseln, Oblongkomprimate, Granulate, Kautabletten, Lutschpastillen, Kaugummi oder Sachets. Diese festen peroral verabreichbaren Formen lassen sich auch als Retard- bzw. Depotsysteme herrichten. Dazu zählen Arzneimittel mit einem Gehalt an einem oder mehreren mikronisierten Wirkstoffen, Diffusions- und Erosionsformen auf Matrixbasis, z.B. unter Verwendung von Fetten, wachsartigen und/oder polymeren Stoffen, oder sogenannten Reservoirsystemen. Wenn das Arzneimittel formuliert wird, um Rizulol über einen längeren Zeitraum freizusetzen können Retardiermittel bzw. Mittel zur gesteuerten Freisetzung, wie film- oder matrixbildende Substanzen, beispielsweise Ethylcellulose, Hydroxpropylmethylcellulose, Poly(met)acrylatderivate (z.B. Eudragit®), Hydroxpropyl-methylcellulosephthalat sowohl in organischen Lösungen als auch in Form wäßriger Dispersionen zugesetzt werden. In diesem Zusammenhang sind auch sogenannte bioadhäsive Präparate zu nennen, bei denen die erhöhte Verweilzeit im Körper durch intensiven Kontakt mit den Körperschleimhäuten erreicht wird. Ein Beispiel eines bioadhäsiven Polymers ist z.B. die Gruppe der Carbomere®.

Zum Zwecke einer gezielten Freisetzung von Riluzol oder eines pharmazeutisch annehmbaren Salzes davon in den verschiedenen Abschnitten des Gastrointestinaltraktes lassen sich Mischungen aus an den verschiedenen Orten freisetzenden Pellets einsetzen. Beispielsweise können Arzneimittelzubereitungen mit Gemischen aus magensaftlöslichen bzw. magensaftresistenten und dickdarmlöslichen Filmen, Stoffen, Verbindungen oder Zusammensetzungen überzogen werden. Dasselbe Ziel der Freisetzung an verschiedenen Abschnitten des Gastrointestinaltraktes läßt sich auch durch entsprechend hergestellte Manteltabletten mit Kern erreichen, wobei der Mantel den Wirkstoff im Magensaft schnell freisetzt und der Kern den Wirkstoff im Dünndarmmilieu allmählich freigibt. Das Ziel einer gesteuerten Freisetzung an verschiedenen Abschnitten des Gastrointestinaltraktes läßt sich beispielsweise auch durch Mehrschichttabletten erreichen. Die Pelletgemische mit unterschiedlich freisetzbarem Wirkstoff lassen sich beispielsweise in Hartgelatinekapseln abfüllen.

Als weitere Hilfsstoffe zur Herstellung von Komprimaten, wie z.B. Tabletten oder Hart- und Weichgelatinekapseln sowie Dragees und Granulaten werden beispielsweise Gegenklebe- und Schmier- und Trennmittel, Dispergiermittel, wie z.B. flammendisperses Siliziumdioxid, Sprengmittel, wie z.B. verschiedene Stärkearten, PVP, Celluloseester als Granulier- oder Retardiermittel, wie z.B. wachsartige und/oder polymere Stoffe auf Eudragit®-, Cellulose- oder Cremophor®- Basis eingesetzt.

Des weiteren können für die perorale Verabreichung zubereitete Arzneimittel Antioxidantia, Süßungsmittel, wie z.B. Saccharose, Xylit oder Mannit, Geschmackskorrigenzien, Aromastoffe, Konservierungsmittel, Farbstoffe, Puffersubstanzen, Direkttablettiermittel, wie z. B. mikrokristalline Cellulose, Stärke und Stärkehydrolysate (z.B. Celutab®), Milchzucker, Polyethylenglykole, Polyvinylpyrrolidon und Dicalciumphosphat, Gleitmittel, Füllstoffe, wie z.B. Lactose oder Stärke, Bindemittel in Form von Lactose, Stärkearten, wie z.B. Weizen- oder Mais- bzw. Reisstärke, Cellulosederivate, wie z.B. Methylcellulose, Hydroxypropylcellulose oder Kieselerde, Talkum, Stearate, wie z.B. Magnesiumstearat, Calciumstearat, Talk, silikonisierter Talk, Stearinsäure, Cetylalkohol oder hydrierte Fette usw. enthalten. Dem Fachmann ist eine Vielzahl von Stoffen bekannt, die Arzneimitteln für die Herrichtung zur perioralen Verabreichung zugesetzt werden können.

In einer weiteren Ausführungsform kann Riluzol oder ein therapeutisch annehmbares Salz davon auch als orales therapeutisches System, insbesondere aufbauend auf osmotischen Prinzipien, wie z.B. GIT gastrointestinales therapeutisches System) oder OROS (orales osmotisches System) formuliert werden.

Zu den peroral verabreichbaren Komprimaten zählen auch Brausetabletten oder Tabs, welche beide rasch in Wasser lösliche oder suspendierbare und sofort trinkbare Instantarzneiformen darstellen.

Zu den peroral verabreichbaren Formen zählen auch Lösungen, z.B. Tropfen, Säfte und Suspensionen, die nach im Stand der Technik bekannter Verfahren hergestellt werden können und - neben den bereits erwähnten Hilfs- und Zusatzstoffen zur Erhöhung der Stabilität - noch Konservierungsmittel und gegebenenfalls zur erleichterten Einnahme noch Aromastoffe und zur besseren Unterscheidbarkeit Farbstoffe sowie Antioxidantia und/oder Vitamine und Süßstoffe, wie Zucker oder künstliche Süßungsmittel enthalten können. Dies gilt auch für Trockensäfte, die vor der Einnahme mit Wasser zubereitet werden. In einer bevorzugten Ausführungsform ein Formulierung des erfindungsgemäßen Arzneimittels in einer flüssig einzunehmenden Form kann diese auch Ionenaustauscherharze enthalten.

Eine spezielle Abgabeform besteht in der Errichtung von sog. Schwimmarzneiformen, beispielsweise auf Basis von Tabletten oder Pellets, die nach Kontakt mit Körperflüssigkeiten Gase entwickeln und deshalb an der Oberfläche der Magenflüssigkeit schwimmen. Weiterhin können auch sogenannte elektronisch gesteuerte Abgabesysteme formuliert werden, bei denen die Wirkstoffabgabe über externe elektronische Impulse gezielt auf die individuellen Bedürfnisse eingestellt werden können.

Eine weitere Gruppe systemisch verabreichbarer und gegebenenfalls auch topisch wirksamer Arzneiformen stellen rektal applizierbare Arzneimittel dar. Dazu zählen die Suppositorien und Klistierzubereitungen. Die Klistierzubereitungen können auf Basis von Tabletten mit wäßrigen Lösungsmitteln zum Herstellen dieser Verabreichungsform hergerichtet werden. Auf der Grundlage von beispielsweise Gelatine oder anderen im Stand der Technik bekannten Trägerstoffen lassen sich auch Rektalkapseln bereitstellen.

Als Suppositoriengrundlagen kommen Hartfette, wie z.B. Witepsol®, Massa Estarium®, Novata®, Kokosfett, Glycerin/Gelatine-Massen, Glycerin/Seifen-Gele und Polyethylenglykole in Betracht.

Für die Langzeitapplikation mit einer systemischen Wirkstoffabgabe bis zu mehreren Wochen sind Implantatpresslinge geeignet, die vorzugsweise auf Basis sog. bioabbaubarer Polymere formuliert sein können.

Das Arzneimittel, das das erfindungsgemäß formulierte Riluzol oder ein pharmazeutisch annehmbares Salz davon enthält, kann auch als transdermales System formuliert werden. Diese Formulierung zeichnet sich wie die obenstehend genannten rektalen Formen durch die Umgehung des Leberkreislaufs bzw. des Lebermetabolismus aus. Als transdermale Systeme sind insbesondere Pflaster geeignet, die auf Basis verschiedener Schichten und/oder Mischungen geeigneter Hilfs- und Trägerstoffe den Wirkstoff in gesteuerter Weise über längere oder kürzere Zeiträume abzugeben vermögen. Bei der Herstellung derartiger transdermaler Systeme können zur Verbesserung und/oder Beschleunigung der Penetration der Haut Substanzen zugesetzt werden, die die Membranendurchdringung erhöhen bzw. Permeationspromotoren, wie z.B. Ölsäure, Azone®, Adipinsäurederivate, Ethanol, Harnstoff, Propylenglykol. Als weitere Bestandteile des erfindungsgemäß verwendbaren Arzneimittels kommen neben geeigneten Hilfs- und Trägerstoffen wie Lösungsmitteln, polymere Bestandteile, z.B. auf Basis von Eudragit®, in Betracht.

Bekannte Formulierungen von Riluzol, die für die orale Administration geeignet sind, sind beispielsweise in EP 0 558 861 und US 4,370,338 offenbart.

Die jeweils für die verschiedenen Applikationswege geeigneten Arzneiformen lassen sich in Einklang mit dem Fachmann bekannten Rezepturvorschriften und Verfahrensweisen auf der Basis allgemein bekannter pharmazeutisch-physikalischer Grundlagen herstellen.

### Kombination mit weiteren Stoffen

In einer weiteren Ausführungsform der vorliegenden Erfindung kann Riluzol oder ein pharmazeutisch annehmbares Salz davon mit anderen therapeutisch aktiven Wirkstoffen kombiniert werden, die zur Behandlung und/oder Prävention von Krankheiten, die durch Hyperproliferation von Keratinozyten gekennzeichnet sind, geeignet sind.

Daher betrifft die vorliegende Erfindung in einem weiteren Aspekt eine Zusammensetzung umfassend Riluzol oder ein pharmazeutisch annehmbares Salz davon und einen oder mehrere weitere Wirkstoffe für die bekannt ist, daß sie zur Therapie oder Prävention von Psoriasis, Neurodermitis, aktinische keratose, Hyperkeratose insbesondere epidermologische Hyperkeratosen, Hyperkeratosis Lenticularis Perstuns, keratosis pilaris und Ichthyosen die durch die Hyperproliferation von Keratinozyten gekennzeichnet sind, verwendet werden können. Besonders geeignete Wirkstoffe, die mit Riluzol oder einem pharmazeutisch annehmbaren Salz davon kombiniert werden können, sind Calcipotriol, Tazarotene, Betamethason, Cortison, Fumarsäure, Clobetasol, Metothrexat, Amphotericin, Busulphan, Co-trimoxazol, Chlorambucil, Colony Stimulating Faktor, Cyclophosphamid, Fluconazol, Ganciclovir, Methylprednisolon, Octreotid, Thalidomid oder Zolimomab aritox.

Eine weitere besonders bevorzugte Ausführungsform betrifft eine Zusammensetzung umfassend Riluzol oder ein pharmazeutsich annehmbares Salz davon und einen Calcineurin-Antagonisten ausgewählt aus Cyclosporin A, Cyclosporin G, Cyclosporin B, Cyclosporin C, Cyclosporin D, Dihydro-cyclosporin D, Cyclosporin E Cyclosporin F, Cyclosporin H, Cyclosporin I, Pimecrolimus und Tacrolimus. Unter dem Begriff "Calcineurin-Antagonist" im Sinne der vorliegenden Erfindung sind Substanzen zu verstehen, die als Antagonist der Calcineurin Phosphatase-Aktivität wirken. Ob eine Substanz antagonistisch auf die Calcineurin Phosphatase-Aktivität wirkt läßt sich durch im Stand der Technik beschriebene Assays zur Bestimmung der Calcineurin Phosphatase-Aktivität bestimmen.. Beispielsweise kann ein Assay durchgeführt werden wie in Baughman et al. (1995, Mol. Cell. Biol., 15: 4395-4402) beschrieben. Der Reaktionsansatz enthält dabei 100 µmol/l CaCl₂, 100 µg Rinderserumalbumin (Fraktion V) pro ml, 40 mmol/l Tris-HCl (pH 8.0), 100 mmol/l NaCl, 6 mmol/l Magnesiumacetat, 500 µmol/l Dithiothreitol, 40 µmol/l [³³P] RII-Peptid (600 cpm/pmol), 190 nmol/l Rinder-Calmodulin, 3 nmol/l Rinder-Calcineurin, 50 µmol/l von der auf Calcineurin-Inhibition zu testende Substanz ("Testsubstanz"), und ein Immunophilin; z.B. FKBP12 and Cyclophilin. Das RII Peptid hat die Sequenz DLDVPIPGRFDRRVSVAAE. Die Phosphorylierung an Serin-Resten wird wie in Liu et al. (1991, Cell, 66: 807-815) und in Manalan und Klee (1983, PNAS, 87:4291-4295) beschrieben, durchgeführt. Die Reaktionsansätze werden 30 min bei 30°C in Abwesenheit markierter Peptide inkubiert. Die Dephosphorylierungsreaktionen werden durch Zugabe der Peptide gestartet und dann für 10 min bei 30°C inkubiert. Die Beendigung der Reaktion sowie die Abtrennung freien Phosphats von phosphorylierten Peptiden erfolgt wie in Liu et. al. und Manalan und Klee (supra) beschrieben. Der Grad der Dephosphorylierung, der in Abwesenheit der Testsubstanz gemessen wird, wird als 100% Calcineurinaktivität definiert, während der Grad der Dephosphorylierung, der in Abwesenheit von Testsubstanz und Calcineurin gemessen wird als 0% Calcineurinaktivität definiert wird. Die Aktivität des jeweiligen Calcineurin-Antagonisten kann dann über die prozentuale Verringerung der Calcineurinaktivität in Gegenwart des jeweiligen Antagonisten ausgedrückt werden. Die Calcineurin-Antagonisten die in der Zusammensetzung der vorliegenden Erfindung verwendet werden verringern die Calcineurinaktivität um mindestens ca. 10%, vorzugsweise um mindestens ca. 30%, bevorzugter um mindestens ca. 50% und am bevorzugtesten um mindestens ca. 90%. Erfindungsgemäße Calcineurin-Antagonisten sind beispielsweise aus WO 95/04061, WO 90/14826, EP 378321, WO 95/09857, WO 95/35299, EP 626385, GB 1491509 und DE2941080 bekannt.

Die erfindungsgemäße Zusammensetzung enthält einen oder mehrere Calcineurin-Antagonisten ausgewählt aus Cyclosporin A, Cyclosporin G, Cyclosporin B, Cyclosporin C, Cyclosporin D, Dihydro-cyclosporin D, Cyclosporin E, Cyclosporin F, Cyclosporin H, Cyclosporin I, Pimecrolimus, Tacrolimus, L-685487 und/oder L-683519. Ganz besonders bevorzugt sind Zusammensetzungen, die neben Riluzol oder einem pharmazeutisch akzeptablen Salz davon Pimecrolimus, Tacrolimus, und Cyclosporin A umfassen. In einer weiteren Ausführungsform können diese Zusammensetzungen auch noch einen oder mehrere der vorgenannten Wirkstoffe und dabei insbesondere einen oder mehrere der besonders geeigneten Wirkstoffe enthalten.

Die erfindungsgemäßen Zusammensetzungen enthaltend einen oder mehrere weitere Wirkstoffe, die die Hyperproliferation von Keratinozyten verringern oder verhindern, und/oder einen oder mehrere Calcineurin-Antagonisten können vom Fachmann in allen vorangehend für Riluzol offenbarten Formulierungen hergestellt und mit den jeweils angegebenen Hilfs- und Zusatzstoffen versetzt werden.

Daher ist ein weiterer Aspekt der vorliegenden Erfindung die Verwendung einer der vorgenannten Zusammensetzungen zur Herstellung eines Arzneimittels zur Therapie oder Prävention von Psoriasis, Neurodermitis, aktinische keratose, Hyperkeratose insbesondere epidermologische Hyperkeratosen, Hyperkeratosis Lenticularis Perstuns, Keratosis pilaris und Ichthyosen, die durch Hyperproliferation von Keratinozyten gekennzeichnet sind, insbesondere von Neurodermitis und Psoriasis. Bei der erfindungsgemäßen Verwendung der erfindungsgemäßen Zusammensetzungen sind die gleichen Applikationsformen wie vorangehend für Riluzol allein erläutert, geeignet, insbesondere die topische Applikation auf die betroffenen Hautstellen.

In einem weiterem Aspekt umfaßt die Erfindung auch räumlich und/oder zeitlich getrennt Verabreichung der jeweiligen Wirkstoffe, d.h. Riluzol; ausgewählt aus Cyclosporin A, Cyclosporin G, Cyclosporin B, Cyclosporin C, Cyclosporin D, Dihydro-cyclosporin D, Cyclosporin E Cyclosporin F, Cyclosporin H, Cyclosporin I, Pimecrolimus und Tacrolimus; und/oder Calcipotriol, Tazarotene, Betamethason, Cortison, Fumarsäure, Clobetasol, Metothrexat, Amphotericin, Busulphan, Co-trimoxazol, Chlorambucil, Colony Stimulating Faktor, Cyclophosphamid, Fluconazol, Ganciclovir, Methylprednisolon, Octreotid, Thalidomid oder Zolimomabaritox.

### Dosis

Die einzusetzende Dosis hängt von der jeweiligen Krankheit und der Schwere der Krankheit ab und liegt im Ermessen des Arztes. Die erfindungsgemäß verwendbaren Arzneimittel enthalten vorzugsweise zwischen ungefähr 0,01 bis ungefähr 500 mg Wirkstoff pro Dosis, vorzugsweise zwischen ungefähr 1 bis ungefähr 100 mg Wirkstoff pro Dosis. Der Wirkstoff kann in einer oder mehreren Dosen pro Tag gegeben werden; alternativ kann der Wirkstoff auch in größeren Zeitabständen gegeben werden.

Im Falle der *in vitro* Messungen (Beispiel 1) wurde ein inhibitorische Wirkung von Riluzol auf die Proliferation bereits ab einer Konzentration von Riluzol von 1 µmol/l gemessen. Je nach Permeabilität der Haut, der Art und Schwere der Krankheit und abhängig von der Art der Formulierung und Häufigkeit der Auftragung können bei topischer Applikation unterschiedliche Konzentrationen an Wirkstoff im Medikament ausreichen, um einen therapeutischen Effekt zu erzielen, vorzugsweise liegt die Konzentration von Riluzol oder ein pharmazeutisch annehmbares Salz davon in einem erfindungsgemäß verwendbaren Arzneimittel zwischen 1 µmol/l und 100 mmol/l.

Daher ist in einer weiteren Ausführungsform der vorliegenden Erfindung ein erfindungsgemäß verwendbares Arzneimittel insbesondere zur topischen Anwendung dadurch gekennzeichnet, daß es Riluzol oder ein pharmazeutisch annehmbares Salz davon in einer Konzentration zwischen 1 µmol/l 1und 100 mmol/l enthält.

### Figuren

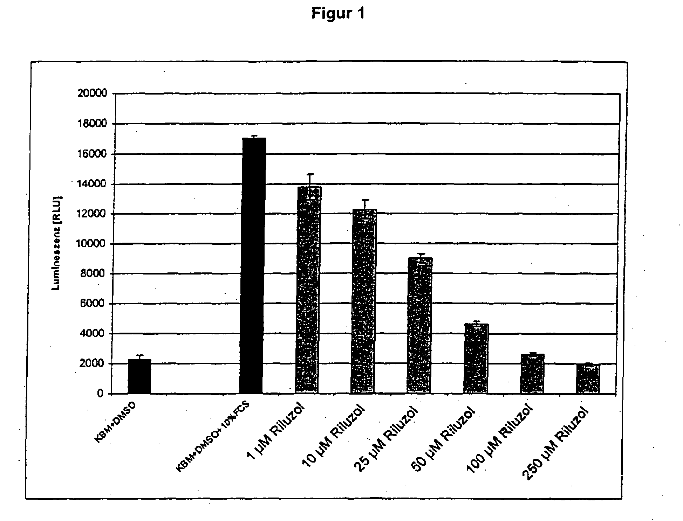

Figur 1: Einfluss von Riluzol auf die Proliferation von Keratinozyten. HaCat-Keratinozyten wurden mit verschiedenen Riluzol-Konzentrationen behandelt (1 µmol/l, 10 µmol/l, 25 µmol/l, 50 µmol/l, 100 µmol/l, 250 µmol/l). Bereits ab 1 µmol/l Riluzol wird eine Inhibition der Proliferation gegenüber der Kontrolle (KBM + 10% FCS) beobachtet. Als weitere Kontrolle dienen Zellen die nur in KBM (KBM) inkubiert wurden.

### Beispiele

### Herstellung von Riluzol

Die Herstellung von Riluzol ist im Stand der Technik beschrieben. So kann Riluzol beispielsweise wie in Yagupol'-skii, L. M. und Gandel'sman L. Z. (Zh. Obshch. Khim., 1963, 33: 2301), US 4,370,338, Jimonet et al. (J. Med. Chem., 1999, 2828-2843) oder Hays et al. (J. Pharm Sci., 1994, 83: 1425-1432) beschrieben hergestellt werden.

### Beispiel 1: Einfluss von Riluzol auf die Keratinozytenproliferation

Es wurde der Einfluss von Riluzol auf die Keratinozytenproliferation anhand von HaCaT-Zellen untersucht. Dazu wurden 5x10³ HaCaT-Keratinozyten in 60 Vertiefungen einer 96 Vertiefungsplatte in jeweils 200 µl KBM/10% FCS ausgesät und für 24 Stunden bei 37°C inkubiert. Nach den Inkubation wurden jeweils 6 Vertiefungen mit HaCaT-Zellen und 1 Vertiefung ohne Zellen für 48 Stunden mit Negativkontrolle (KBM/1% DMSO), Positivkontrolle (KBM/FCS/1% DMSO) oder mit 0,1 - 250 µmol/l Riluzol in KBM/FCS (Stammlösung Riluzol: 100 mmol/l in DMSO) behandelt und für 48 Stunden bei 37°C inkubiert. Dabei wurde die DMSO-Konzentration in allen getesteten Riluzolkonzentrationen konstant bei 1 % gehalten. Am Ende der 2. Inkubationsphase wurde das Medium entfernt und die Proliferation der Zellen mit dem Cell Titer Viability Assay von Promega (#G7571/G7572) nach den Anweisungen des Herstellers bestimmt. Dabei korreliert eine gegenüber der Positivkontrolle erniedrigte Lumineszenz (in RLU, relative Lumineszenzeinheiten) mit einer erniedrigten Proliferation. Werte, die von den Vertiefungen ohne Zellen erhalten wurden, wurden als Hintergrundwerte von dem Mittelwert der 6 Vertiefungen mit Zellen abgezogen. Es wurden mindestens 4 unabhängige Experimente durchgeführt. Die Ergebnisse sind in Figur 1 zusammengestellt. Es zeigt sich, daß Riluzol bereits im niedrigen µmol/l-Bereich die Proliferation von Keratinozyten hemmt und im höheren µmol/l-Bereich (100 µmol/l, 250 µmol/l) eine völlige Inhibition der Proliferation erreicht wird; d.h. ein Wert vergleichbar mit der Negativkontrolle erreicht wird. Dies zeigt die besondere Eignung von Riluzol zur Therapie oder Prävention von Erkrankungen, die durch Hyperproliferation von Keratinozyten gekennzeichnet sind, insbesondere zur Therapie von Psoriasis.

### Beispiel 2: Einfluss von Riluzol auf den psoriatrischen Phänotyp im Psoriasis-Tiermodell

Die Wirkung von Riluzol kann beispielsweise im SCID-Maus Tiermodell nachgewiesen werden, z.B. wie in Boehncke et al. (Arch. Dermatol. Res. 286:325-330) beschrieben. Dazu werden spindelförmige Hautbiopsien von Läsionen von Psoriasispatienten entnommen, und Transplantate von 1 cm Durchmesser auf ebenso große Wunden auf dem Rücken von SCID-Mäusen transplantiert. Danach wird ca. 2 Wochen gewartet bis das Gewebe angewachsen ist.

Anschließend kann die transplantierte Biopsie mit Riluzol, das in Basiscreme formuliert ist, topisch appliziert oder als Lösung intradermal gespritzt werden. Beispielsweise kann Riluzol in Konzentrationen von 1 µmol/l, 10 µmol/l, 100 µmol/l, 1 mmol/l, 10 mmol/l, 20 mmol/l, 100 mmol/l getestet werden.

Die Creme oder die Lösung werden täglich 1-2 mal appliziert. Nach 3 Behandlungswochen werden die Tiere getötet, die Biopsien entnommen und histologisch untersucht. Die Biopsien werden dazu mit Standard-Eosin- und -Hämatoxylin-Färbungen gefärbt und auf Veränderungen der Dicke der Epidermis hin untersucht. Eine Verringerung der Epidermisdicke im Vergleich zu Kontrollmäusen, die mit Trägersubstanz alleine behandelt wurde, weist die Wirksamkeit von Riluzol im Tiermodell nach.

## Patentansprüche

1. Verwendung von Riluzol oder eines pharmazeutisch annehmbaren Salzes davon gegebenenfalls mit geeigneten Hilfs- und Zusatzstoffen zur Herstellung eines Arzneimittels zur Therapie oder Prävention von Psoriasis, Neurodermitis, aktinischer Keratose, Hyperkeratosen wie epidermolytische Hyperkeratose, Hyperkeratosis Lenticularis Perstans, Keratosis pilaris und Ichthyosen.

2. Verwendung nach Anspruch 1, **dadurch gekennzeichnet, daß** das Arzneimittel topisch appliziert wird.

3. Verwendung nach Anspruch 2, **dadurch gekennzeichnet, daß** das Arzneimittel in Form einer Salbe, eines Gels, eines Pflasters, einer Emulsion, einer Lotion, eines Schaums, einer Creme eines mischphasigen oder amphiphilem Emulsionssystems (Öl/ Wasser-Wasser/Öl-Mischphase), eines Liposoms, eines Transfersoms, einer Paste oder eines Puders zubereitet ist.

4. Verwendung nach einem der Ansprüche 1 bis 3, **dadurch gekennzeichnet, daß** Riluzol oder das pharmazeutisch annehmbare Salze davon in dem Arzneimittel in einer Konzentration zwischen 1 µmol/l und 100 mmol/l enthalten ist.

5. Zusammensetzung umfassend Riluzol oder ein pharmazeutisch annehmbares Salz davon und einen oder mehrere Wirkstoffe, ausgewählt aus Calcipotriol, Tazarotene, Betamethason, Cortison, Fumarsäure, Clobetasol, Metothrexat Amphotericin, Busulphan, Co-trimoxazol, Chlorambucil, Colony Stimulating Faktor, Cyclophosphamid, Fluconazol, Ganciclovir, Methylprednisolon, Octreotid, Thalidomid, Zolimomab aritox., Cyclosporin A, Cyclosporin G, Cyclosporin B, Cyclosporin C, Cyclosporin D, Dihydro-cyclosporin D, Cyclosporin E, Cyclosporin F, Cyclosporin H, Cyclosporin I, Pimecrolimus und Tacrolimus.

6. Verwendung einer Zusammensetzung nach Anspruch 5 gegebenenfalls mit geeigneten Hilfs- und Zusatzstoffen zur Herstellung eines Arzneimittels zur Therapie oder Prävention von Psoriasis, Neurodermitis, aktinischer Keratose, Hyperkeratosen wie epidermolytische Hyperkeratose, Hyperkeratosis Lenticularis Perstans, Keratosis pilaris und Ichthyosen.

## Claims

1. Use of riluzol or a pharmaceutical acceptable salt thereof, optionally with appropriate auxiliary substances and additives for the production of a medicament for the therapy or prevention of psoriasis, neurodermatitis, actinic ceratosis, hyperceratosis with epodermolytic hyperceratosis, hyperceratosis lenticularis perstans, ceratosis pilaris and ichthyosis.

2. Use according to claim 1, **characterized in that that** the medicament is applied topically.

3. Use according to claim 2, **characterized in that** the medicament is in the form of an ointment, a gel, a band-aid, an emulsion, a lotion, a foam, a crème of a mixed phase or an amphiphilic emulsion system (oil/water-water/oil-mixed phase), a liposome, a transferosome, a paste or a powder.

4. Use according to one of claims 1 to 3, **characterized in that that** riluzol or a pharmaceutically acceptable salt thereof is comprised in the medicament in the concentration between 1 µmol/l and 100 mmol/l.

5. Composition comprising riluzol or a pharmaceutically acceptable salt thereof and one or more active ingredients selected from calcipotriole, tazarotene, betamethasone, cortisone, fumaric acid, clobetasole, metothrexate, amphotericine, busulphane, co-trimoxazole, chloro-ambucile, colony stimulating factor, cyclophosphamide, fluconazole, ganciclovir, methylprednisolone, octreotide, thalidomide, zolimomab aritox., cyclosporine A, cyclosporine G, cyclosporine B, cyclosporine C, cyclosporine D, dihydro-cyclosporine D, cyclosporine E, cyclosporine F, cyclosporine H, cyclosporine I, pimecrolimus and tacrolimus.

6. Use of a composition according to claim 5 optionally with appropriate auxiliary substances and additives for the production of the medicament for a therapy or prevention of psoriasis, neurodermatitis, actinic ceratosis, hyperceratosis with epodermolytic hyperceratosis, hyperceratosis lenticularis perstans, ceratosis pilaris and ichthyosis.

## Revendications

1. Utilisation de rilusole ou d'un de ses sels acceptables d'un point de vue pharmaceutique et éventuellement avec des excipients et des additifs pour fabriquer un médicament pour la thérapie ou la prévention du psiorasis, de la névrodermite, de la kératose actinique, de l'hyperkératose comme l'hyperkératose épidermolytique, l'hyperkératose lenticularis perstans, la kératose pilaris et les ichtyoses.

2. Utilisation selon la revendication 1, **caractérisé que** le médicament est appliqué de façon topique.

3. Utilisation selon la revendication 2, **caractérisé que** le médicament est préparé sous la forme d'une pommade, d'un gel, d'un patch, d'une émulsion, d'une lotion, d'une mousse, d'une crème d'un système d'émulsion à phases mélangés ou amphiphiles (phase mélangé huile / eau - eau /huile), d'un liposome, d'un transfersome, d'une pâte ou d'un poudre.

4. Utilisation selon une des revendications 1 à 3, **caractérisé en ce que** le rilusole ou un de ses sels acceptables d'un point de vue pharmaceutique est contenu dans le médicament avec une concentration entre 1 µmol/l et 100 µmol/l.

5. Composition comportant le rilusole ou un de ses sels acceptables d'un point de vue pharmaceutique ou un ou plusieurs agents actifs, choisis parmi les substances calcipotriole, tazarotène, bétamethasone, cortisone, acide fumarique, clobétasole, métothrexate amphotéricine; busulphane, co-trimoxazole, chlorambucile, facteur de stimulation des colonies, cyclophosphamide, fluconazole, ganciciovire, méthylprednisolone, octréotide, thalidomide, zolimomab aritox, cyclosporine A, cyclosporine G, cyclosporine B, cyclosporine C, cyclosporine D, dihydro-cyclosporine D, cyclosporine E, cyclosporine F, cyclosporine H, cyclosporine I, pimécrolimus et tacrolimus.

6. Utilisation d'une composition selon la revendication 5 , éventuellement avec des excipients et des additifs pour fabriquer un médicament pour la thérapie ou la prévention du psiorasis, de la névrodermite, de la kératose actinique, de l'hyperkératose comme l'hyperkératose épidermolytique, l'hyperkératose lenticularis perstans, la kératose pilaris et les ichtyoses.
